(19) Europäisches Patentamt
European Patent Office
Office européen des brevets

(11) Veröffentlichungsnummer: **0 377 198 A2**

# (12) EUROPÄISCHE PATENTANMELDUNG

(21) Anmeldenummer: **89123924.6**

(22) Anmeldetag: **27.12.89**

(51) Int. Cl.5: **C07C 69/96, C07C 68/02, C07D 335/16**

(30) Priorität: **31.12.88 DE 3844443**

(43) Veröffentlichungstag der Anmeldung: **11.07.90 Patentblatt 90/28**

(84) Benannte Vertragsstaaten: **AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Boettcher, Andreas, Dr.**
**Konrad-Adenauer-Ring 38**
**D-6907 Nussloch(DE)**
Erfinder: **Henkelmann, Jochem, Dr.**
**Volkerstrasse 26**
**D-6140 Bensheim(DE)**

## (54) Chlorformiate aromatischer Ketone und Verfahren zu deren Herstellung.

(57) Die Erfindung betrifft Chlorformiate aromatischer Ketone sowie deren Herstellung aus aromatischen Hydroxyketonen durch Phosgenierung in Gegenwart saurer Katalysatoren.

Die erfindungsgemäßen Chlorformiate sind Zwischenprodukte für die Synthese von organischen Verbindungen.

EP 0 377 198 A2

Xerox Copy Centre

## Chlorformiate aromatischer Ketone und Verfahren zu deren Herstellung

Die vorliegende Erfindung betrifft neue Chlorformiate aromatischer Ketone sowie ein verbessertes Verfahren zur Herstellung von aromatischen Chlorformiaten durch Phosgenierung von Hydroxyaromaten in Gegenwart katalytischer Mengen eines sauren Katalysators.

Die Herstellung der für zahlreiche organische Synthesen wichtigen Chlorformiate, die auch als Chlorocarbonate oder Ester der Chlorkohlensäure bezeichnet werden, durch Phosgenierung entsprechender Phenole ist allgemein bekannt (vgl. z.B. Houben-Weyl, Methoden der Organischen Chemie Bd. 8, S. 75, 101-107, Thieme Verlag 1952; J. Prakt. Chem. 313, 331 (1971); J. Prakt. Chem. 317, 62, 73, 81 (1975)).

Da diese Reaktion bei Phenolen relativ langsam und häufig auch nur unvollständig verläuft, wurde verschiedentlich empfohlen, sie in Gegenwart von Katalysatoren vorzunehmen.

Speziell aus der DE-A-27 40 248 und der US-A-3 299 114 ist bekannt, als Katalysatoren offenkettige oder heterocyclische tertiäre Alkyl- oder Arylamine zu verwenden. Dieser Syntheseweg ist unbefriedigend, weil nicht nur z.T. mäßige Ausbeuten an Chlorformiaten der zur Diskussion stehenden Art erzielt werden, sondern auch die verwendeten Katalysatoren zu verfahrenstechnischen Schwierigkeiten Anlaß geben, weil die aus ihnen bei der Phosgenierung gebildeten Hydrochloride im Reaktionsgemisch unlöslich sind und somit zusätzliche Trennoperationen erforderlich machen. Weiterhin sind die nach Abschluß der Reaktion erhaltenen Hydrochloride für einen erneuten Einsatz unbrauchbar, da sie desaktiviert sind.

Der Erfindung lag daher die Aufgabe zugrunde, diesen Nachteilen abzuhelfen.

Gegenstand der vorliegenden Erfindung sind Chlorformiate aromatischer Ketone der allgemeinen Formel (I)

$$R-\overset{\overset{O}{||}}{C}-R^1 \quad \text{(I)},$$

worin

R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest, für einen Aroylrest oder den Rest $R^1$ steht und

$R^1$ für den Rest

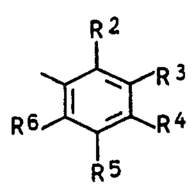

steht, wobei die Reste

$R^2$ bis $R^6$ untereinander gleich oder verschieden sind und für H, Alkyl mit 1 bis 4 Kohlenstoffatomen, OH, $OCH_3$, $OC_2H_5$, SH, $SCH_3$, $SC_2H_5$, F, Cl, Br, CN, COOAlkyl mit Alkyl, enthaltend 1 bis 17 Kohlenstoffatome, COOAryl, $CF_3$, $N(Alkyl)_2$, N(Alkyl)-(Aryl), $N(Aryl)_2$, $N^{\oplus}(Alkyl)_3A^{\ominus}$, $N^{\oplus}H(Alkyl)_2A^{\ominus}$ mit Alkyl enthaltend 1 bis 4 Kohlenstoffatome und $A^{\ominus}$ für das Anion einer Säure, z.B. $Cl^{\ominus}$, $SO_4^{2\ominus}$, $PO_4^{3\ominus}$, $Acetat^{\ominus}$, $CF_3SO_3^{\ominus}$, $BF_4^{\ominus}$, $AsF_6^{\ominus}$, $SbF_6^{\ominus}$, $PF_6^{\ominus}$ usw. stehen und mindestens einer aber maximal drei der Reste $R^2$ bis $R^6$ für den Chlorformylrest -OCOCl stehen, oder für den Fall, daß R für einen Arylrest steht, einer der Reste $R^2$ oder $R^6$ für ein Schwefelatom stehen kann, durch das der Arylrest R in ortho-Position unter Ausbildung eines weiteren Ringes mit $R^1$ verbunden ist.

Gegenstand der vorliegenden Erfindung ist außerdem ein Verfahren zur Herstellung von Chlorformiaten, das dadurch gekennzeichnet ist, daß aromatische Hydroxyketone der allgemeinen Formel (II),

$$R-\overset{\overset{O}{||}}{C}-R' \quad \text{(II)},$$

worin

R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest, für einen Aroylrest oder den Rest R' steht und

R' für den Rest

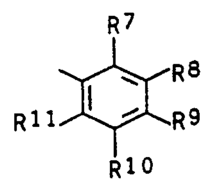

steht, wobei die Reste

$R^7$ bis $R^{11}$ untereinander gleich oder verschieden sind und für H, Alkyl mit 1 bis 4 Kohlenstoffatomen, $OCH_3$, $OC_2H_5$, $SCH_3$, $SC_2H_5$, F, Cl, Br, CN, COOAlkyl mit Alkyl enthaltend 1 bis 17 Kohlenstoffatome, COOAryl, $CF_3$, $N(Alkyl)_2$, N(Alkyl)(Aryl), N-$(Aryl)_2$, $N^{\oplus}(Alkyl)_3A^{\ominus}$, $N^{\oplus}H(Alkyl)_2A^{\ominus}$ mit Alkyl enthaltend 1 bis 4 Kohlenstoffatome und $A^{\ominus}$ für das Anion einer Säure, z.B. $Cl^{\ominus}$, $SO_4^{2\ominus}$, $PO_4^{3\ominus}$, $Acetat^{\ominus}$, $CF_3SO_3^{\ominus}$, $BF_4^{\ominus}$, $AsF_6^{\ominus}$, $SbF_6^{\ominus}$, $PF_6^{\ominus}$ usw. stehen und mindestens einer aber maximal drei der Reste $R^7$ bis $R^{11}$ für eine Hydroxygruppe steht, oder für den Fall, daß R für einen Arylrest steht, einer der Reste $R^7$ oder $R^{11}$ für ein Schwefelatom steht, durch das der Arylrest R in ortho-Position unter Ausbildung eines weiteren Ringes

mit R′ verbunden ist, in Gegenwart eines sauren Katalysators, gegebenenfalls in Gegenwart eines inerten Lösungsmittels oder Lösungsmittelgemisches, bei Temperaturen von 50 bis 170 °C unter wasserfreien Bedingungen mit, bezogen auf Hydroxygruppen in R′, äquivalenten Mengen Phosgen oder Phosgen liefernden Verbindungen umgesetzt werden.

Als aromatische Hydroxyketone werden vorzugsweise Hydroxyacetophenone, Hydroxybenzophenone, Hydroxybenzile oder Hydroxythioxanthone eingesetzt.

Für das erfindungsgemäße Verfahren zur Herstellung von aromatischen Chlorformiaten eignen sich beispielsweise Verbindungen der allgemeinen Formel (II)

$$R-\overset{O}{\overset{\|}{C}}-R' \quad \text{(II)},$$

worin

R für einen geradkettigen Alkylrest mit 1 bis 4 Kohlenstoffatomen, vorzugsweise Methyl, Ethyl, n-Propyl, einen verzweigten, gegebenenfalls substituierten, Alkylrest mit 3 oder 4 Kohlenstoffatomen, wie iso-Propyl, sek.-Hydroxyisopropyl, tert.-Butyl, einen Arylrest, wie Phenyl, Tolyl oder Naphthyl, einen Aroylrest, wie z.B. Benzoyl, Toloyl- oder Mesitoyl, oder den Rest R′ steht und

R′ für den Rest

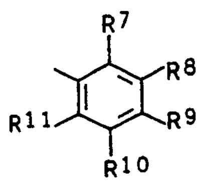

steht, wobei die Reste

$R^7$ bis $R^{11}$ untereinander gleich oder verschieden sind und für H, Alkyl mit 1 bis 3 Kohlenstoffatomen, z.B. Methyl, Ethyl, n-Propyl, iso-Propyl, $OCH_3$, $OC_2H_5$, $SCH_3$, $SC_2H_5$, F, Cl, Br, CN, COOAlkyl mit Alkyl enthaltend 1 bis 17 Kohlenstoffatome, COOAryl, $CF_3$, $N(Alkyl)_2$, N(Alkyl)(Aryl), $N(Aryl)_2$, $N^{\oplus}(Alkyl)_3A^{\ominus}$, $N^{\oplus}H(Alkyl)_2A^{\ominus}$ mit Alkyl enthaltend 1 bis 4 Kohlenstoffatome und $A^{\ominus}$ für das Anion einer Säure, z.B. $Cl^{\ominus}$, $SO_4^{2\ominus}$, $PO_4^{3\ominus}$, $Acetat^{\ominus}$, $CF_3SO_3^{\ominus}$, $BF_4^{\ominus}$, $AsF_6^{\ominus}$, $SbF_6^{\ominus}$, $PF_6^{\ominus}$ usw. stehen und mindestens einer aber maximal drei der Reste $R^7$ bis $R^{11}$ für eine Hydroxygruppe steht, wobei für den Fall, daß R für einen Arylrest steht, einer der Reste $R^7$ oder $R^{11}$ für ein Schwefelatom stehen kann, durch das der Arylrest R in ortho-Position unter Ausbildung eines weiteren Ringes mit R′ verbunden ist.

Überraschenderweise wurde gefunden, daß nicht basisch reagierende Katalysatoren, sondern im Gegenteil saure Katalysatoren besonders geeignet sind. Diese Beobachtung war nicht vorhersehbar, da bei der Phosgenierung in Gegenwart basischer Katalysatoren die Bildung von Hydrochloriden wahrscheinlich ist.

Bevorzugte saure Katalysatoren sind Tetraalkyl, Trialkyl-aryl-, Dialkyl-diaryl-, Alkyl-triaryl-, Tetraarylammonium- und/oder -phosphoniumsalze.

Als Beispiele für die erfindungsgemäß vorzugsweise einzusetzenden Katalysatoren seien genannt:
Heptyl- bis Palmitylbenzyldimethylammoniumchlorid,
Benzyltributylammoniumchlorid,
Benzyltriethylammoniumchlorid,
Benzyltrimethylammoniumchlorid,
Butyltriphenylphosphoniumchlorid,
Ethyltrioctylphosphoniumchlorid,
Hexadecyltrimethylammoniumchlorid,
Methyltrioctylammoniumchlorid,
Tetrabutylammoniumchlorid,
Tetrabutylphosphoniumchlorid,
Tetraethylammoniumchlorid,
Tetraoctylammoniumchlorid,
Tetraphenylphosphoniumchlorid,
Tetrapropylphosphoniumchlorid,
Tributylmethylammoniumchlorid.

Die für das erfindungsgemäße Verfahren bevorzugt einzusetzenden Katalysatoren haben einen sehr schwach ausgeprägten salzartigen Charakter, was für die katalytische Wirkung dieser Basen vorteilhaft ist. Ein Kriterium für die Wirksamkeit dieser Katalysatoren ist auch der große räumliche Bedarf der Alkyl- und/oder Arylreste (vgl. DE-A-37 31 812).

Die erfindungsgemäß einzusetzenden Katalysatoren weisen eine außergewöhnlich hohe katalytische Wirksamkeit auf, die bereits in Mengen von 0,01 mol% pro Hydroxylgruppe des aromatischen Hydroxyketons zu beobachten ist. Vorzugsweise setzt man die Katalysatoren in Mengen von 0,05 bis 0,5 mol%, bezogen auf die Hydroxylgruppe des aromatischen Hydroxyketons, ein.

Oberhalb von 1 mol% ergeben sich kaum noch wirtschaftliche Vorteile, so daß man nur in Ausnahmefällen, etwa zur Beschleunigung der Umsetzung von sehr reaktionsträgen aromatischen Hydroxyketonen größere Mengen - etwa bis zu 10 mol% - verwenden wird.

Da die Menge der Katalysatoren normalerweise nur sehr gering ist, ist es nicht erforderlich, sie von den Verfahrensprodukten abzutrennen, denn bei den weiteren Reaktionen dieser Zwischenprodukte stören die Katalysatoren zumindest in geringen Konzentrationen im allgemeinen nicht. Hierin liegt ein weiterer Vorteil der erfindungsgemäß zu verwendeten Katalysatoren.

Sieht man von den speziellen Katalysatoren ab, gleicht die Phosgenierung derjenigen des Standes der Technik, wobei allerdings die hohe Wirksamkeit der Katalysatoren schonendere Bedingungen gestattet.

Für die Reaktionstemperaturen kommt im allgemeinen der Bereich von 20 bis 200 °C in Betracht, wobei Temperaturen zwischen 50 und 170 °C, insbesondere zwischen 90 und 130 °C, bevorzugt sind.

Man führt die Umsetzung vorzugsweise unter Normaldruck aus, jedoch kann man auch bei höherem Druck - etwa bis zu 10 bar - arbeiten, z.B. wenn das aromatische Hydroxyketon leicht flüchtig ist.

Die Mitverwendung von Lösungsmitteln ist im allgemeinen nicht erforderlich, es sei denn, das eingesetzte aromatische Hydroxyketon und/oder das erfindungsgemäße Verfahrensprodukt wären unter den Reaktionsbedingungen nicht flüssig. Als Lösungsmittel oder auch als Medien für Reaktionen in Suspension eignen sich inerte organische Flüssigkeiten wie aliphatische und aromatische Kohlenwasserstoffe, beispielsweise Hexan, Toluol und Xylol, chlorierte Kohlenwasserstoffe wie Methylenchlorid und Chlorbenzol, Ether wie Dioxan und Tetrahydrofuran und Lactame wie N-Methylpyrrolidon. Vorteilhafterweise können die erfindungsgemäßen Chlorformiate dabei auch als Lösungsmittel dienen.

Im übrigen bietet die erfindungsgemäße Phosgenierung, die man wie üblich diskontinuierlich, halbkontinuierlich oder kontinuierlich ausführen kann, keine verfahrenstechnischen oder apparativen Besonderheiten, so daß sich nähere Ausführungen hierzu erübrigen. Das gleiche gilt für die Aufarbeitung der Reaktionsgemische.

Die als Ausgangsmaterial benötigten Hydroxyacetophenone und Hydroxybenzophenone sind nach bekannten Verfahren herstellbar. So erhält man beispielsweise 4-Hydroxybenzophenon in ca. 90 %iger Ausbeute durch Friedel-Crafts-Acylierung von Phenol mit Benzoylchlorid in Nitrobenzol in Gegenwart von $AlCl_3$ oder $TiCl_4$ (vgl. z.B. Houben-Weyl, Methoden der organischen Chemie, Band 7/2a, S. 186) oder isomerenfrei durch Oxidation von 4-Hydroxy-diphenylmethan mit 5,6-Dichlor-2,3-dicyan-p-benzochinon (vgl. z.B. Houben-Weyl, Band 7/2a, S. 681).

Das 2-Hydroxythioxanthon kann nach dem in GB-PS 2 108 487 und GB-PS 2 108 979 beschriebenen Verfahren aus Thiosalicylsäure und Phenol dargestellt werden.

(4-Hydroxyphenyl)-2-hydroxy-2-propylketon ist in DE-A-35 34 645 beschrieben.

Als charakteristische Beispiele für die erfindungsgemäß einzusetzenden Phenole seien genannt:

4-Hydroxybenzil
4-Chlor-5′-fluor-2′-hydroxybenzophenon
4-Chlor-4′-hydroxybenzophenon
2,4-Dihydroxybenzophenon
4,4′-Dihydroxybenzophenon
4-Fluor-4′-hydroxybenzophenon
2-Hydroxybenzophenon
4-Hydroxybenzophenon
2-Hydroxy-4-methoxybenzophenon
2,3,4-Trihydroxybenzophenon
(4-Hydroxyphenyl)-(2-hydroxy-2-propyl)keton
2-Hydroxy-thioxanthon
3-Hydroxy-thioxanthon

Die erfindungsgemäßen Chlorformiate können im Labormaßstab auch unter Verwendung von Phosgen-Alternativen, wie dem Trichlormethylchlorformiat (Diphosgen), J. Prakt. Chem. 126, 210 (1930), dito 128, 233 (1930), Chem. Abstr. 95, 81766, J. Org. Chem. 50, 715 (1985), J. Org. Chem. 41, 2070 (1976); Angew. Chem. 89, 267 (1977) oder dem kristallinen Triphosgen, Angew. Chem. 99, 922 (1987) in guten Ausbeuten hergestellt werden.

Die erfindungsgemäßen Chlorformiate sind Zwischenprodukte, u.a. für die Synthese von organischen Verbindungen, die als Arznei- und Pflanzenschutzmittel, sowie als Additive für Polymere und Photopolymere dienen.

Für alle in den folgenden Beispielen angegebenen Verbindungen wurde die Struktur z.T. durch unabhängige Synthesen, d.h. durch alternative Phosgenierung mit Di- und Triphosgen und in allen Fällen durch korrekte $^1$H-NMR-, IR- und Massenspektren sowie durch übereinstimmende Elementaranalysen bestätigt. Die in den Beispielen angegebenen Prozente sind Gewichtsprozente.

Beispiel 1

4-Chlorformylbenzophenon

In eine Lösung von 4 kg 4-Hydroxybenzophenon und 190 g Benzyltrimethylammoniumchlorid in 11,5 kg o-Xylol wurden innerhalb von 5 Stunden insgesamt 3,4 kg Phosgen eingeleitet. Die Innentemperatur wurde während dieser Zeit von 95 auf 120 °C erhöht. Nach Beendigung der Phosgeneinleitung wurde 30 Minuten bei 115 °C nachgerührt. Zur Aufarbeitung wurde der Phosgenüberschuß mit Stickstoff ausgetrieben. Das gegen Ende der Reaktion ausgefallene Salz wurde abfiltriert und das Lösungsmittel abdestilliert. Man erhielt 4,9 kg (93 %) gelblich gefärbtes 4-Chlorformylbenzophenon vom Schmp. 67 - 72 °C. Dieses Rohprodukt mit Cl-Wert = 12,69 % (theor. 13.60 %) ließ sich direkt ohne weitere Reinigung für die Folgereaktionen verwenden. Das Rohprodukt läßt sich nach üblichen Methoden, wie z.B. durch Umkristallisieren aus aprotischen Lösungsmitteln, wie z.B. Toluol, Acetonitril und/oder Essigester reinigen.

In Analogie zu der im Beispiel 1 genannten

Vorschrift wurden die folgenden Chlorformylverbindungen hergestellt:

Beispiel 2

Aus Hydroxythioxanthon wurden in 79 %iger Ausbeute 2-Chlorformyl-thioxanthon (Cl: Ber. = 12,19 %; Gef. = 12,03 %) erhalten.

Beispiel 3

3-Chlorformylthioxanthon war in 63 %iger Ausbeute (Cl: Ber. = 12.19 %, Gef. = 11,22 %) zugänglich.

Beispiel 4

Aus (4-Hydroxyphenyl)-(2-hydroxy-2-propyl)-keton wurden in 75 %iger Ausbeute (4-Chlorformylphenyl)-2-hydroxy-2-propyl)-keton als Rohprodukt (Cl: Ber. = 14,61 %; Gef. = 13,07 %) erhalten.

**Ansprüche**

1. Chlorformiate aromatischer Ketone der allgemeinen Formel (I)

$$R-\overset{\overset{O}{\|}}{C}-R^1 \quad (I),$$

worin
R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest, für einen Aroylrest oder den Rest $R^1$ steht und
$R^1$ für den Rest

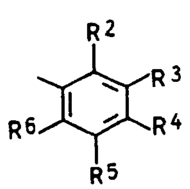

steht, wobei die Reste
$R^2$ bis $R^6$ untereinander gleich oder verschieden sind und für H, Alkyl mit 1 bis 4 Kohlenstoffatomen, OH, $OCH_3$, $OC_2H_5$, SH, $SCH_3$, $SC_2H_5$, F, Cl, Br, CN, COOAlkyl mit Alkyl, enthaltend 1 bis 17 Kohlenstoffatome, COOAryl, $CF_3$, $N(Alkyl)_2$, N(Alkyl)-(Aryl), $N(Aryl)_2$, $N^{\oplus}(Alkyl)_3A^{\ominus}$, $N^{\oplus}H(Alkyl)_2A^{\ominus}$ mit Alkyl, enthaltend 1 bis 4 Kohlenstoffatome, und $A^{\ominus}$ für das Anion einer Säure stehen und mindestens einer aber maximal drei der Reste $R^2$ bis $R^6$ für den Chlorformylrest -OCOCl stehen, oder für den Fall, daß R für einen Arylrest steht, einer der Reste $R^2$ oder $R^6$ für ein Schwefelatom stehen kann, durch das der Arylrest R in ortho-Position unter Ausbildung eines weiteren Ringes mit $R^1$ verbunden ist.

2. Verfahren zur Herstellung von Chlorformiaten nach Anspruch 1, dadurch gekennzeichnet, daß aromatische Hydroxyketone der allgemeinen Formel (II),

$$R-\overset{\overset{O}{\|}}{C}-R' \quad (II),$$

worin
R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest, für einen Aroylrest oder den Rest $R'$ steht und
$R'$ für den Rest

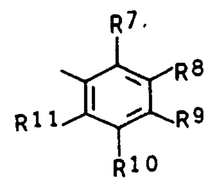

steht, wobei die Reste
$R^7$ bis $R^{11}$ untereinander gleich oder verschieden sind und für H, Alkyl mit 1 bis 4 Kohlenstoffatomen, $OCH_3$, $OC_2H_5$, $SCH_3$, $SC_2H_5$, F, Cl, Br, CN, COOAlkyl mit Alkyl enthaltend 1 bis 17 Kohlenstoffatome, COOAryl, $CF_3$, $N(Alkyl)_2$, N(Alkyl)(Aryl), N-$(Aryl)_2$, $N^{\oplus}(Alkyl)_3A^{\ominus}$, $N^{\oplus}H(Alkyl)_2A^{\ominus}$ mit Alkyl enthaltend 1 bis 4 Kohlenstoffatome und $A^{\ominus}$ für das Anion einer Säure stehen und mindestens einer aber maximal drei der Reste $R^7$ bis $R^{11}$ für eine Hydroxygruppe steht, oder für den Fall, daß R für einen Arylrest steht, einer der Reste $R^7$ oder $R^{11}$ für ein Schwefelatom stehen kann, durch das der Arylrest R in ortho-Position unter Ausbildung eines weiteren Ringes mit $R'$ verbunden ist, in Gegenwart eines sauren Katalysators, gegebenenfalls in Gegenwart eines inerten Lösungsmittels oder Lösungsmittelgemisches, bei Temperaturen von 50 bis 170 °C unter wasserfreien Bedingungen mit, bezogen auf Hydroxygruppen in $R'$, äquivalenten Mengen Phosgen oder Phosgen liefernden Verbindungen umgesetzt werden.

3. Verfahren nach Anspruch 2, dadurch gekennzeichnet, daß als aromatische Hydroxyketone Hydroxyacetophenone, Hydroxybenzophenone, Hydroxybenzile oder Hydroxythioxanthone eingesetzt werden.

4. Verfahren nach Anspruch 2 oder 3, dadurch gekennzeichnet, daß als Phosgen liefernde Verbindungen Diphosgen oder Triphosgen verwendet werden.

5. Verfahren nach einem der Ansprüche 2 bis 4, dadurch gekennzeichnet, daß als saure Katalysatoren Tetraalkyl-, Trialkyl-aryl, Dialkyl-diaryl-, Alkyl-triaryl-, Tetraarylammonium- und/oder -phosphoniumsalze verwendet werden.